## Europäisches Patentamt

## European Patent Office

## Office européen des brevets

(19)

(11) Veröffentlichungsnummer: **0 011 665**
A1

(12) **EUROPÄISCHE PATENTANMELDUNG**

(21) Anmeldenummer: **78810026.1**

(22) Anmeldetag: **23.11.78**

(51) Int. Cl.³: **A 61 F 1/00**

(43) Veröffentlichungstag der Anmeldung:
**11.06.80 Patentblatt 80/12**

(84) Benannte Vertragsstaaten:
**BE CH DE FR GB LU NL SE**

(71) Anmelder: **OSTEO AG**
**Bielstrasse 620**
**CH-2545 Selzach(CH)**

(72) Erfinder: **Mittelmeier, Heinz, Prof. Dr. med.**
**An der Schutzhütte**
**Blieskastel(DE)**

(72) Erfinder: **Moser, Heinz**
**Späretweg 440**
**CH-2545 Selzach(CH)**

(72) Erfinder: **Leu, Beat**
**Hintere Gasse 33**
**CH-2500 Biel(CH)**

(74) Vertreter: **Seehof, Michel et al,**
**c/o AMMANN PATENTANWAELTE AG BERN**
**Schwarztorstrasse 31**
**CH-3001 Bern(CH)**

(54) Satz von Endoprothesenteilen und Verfahren zur Anpassung der Halslänge dieser Endoprothese.

(57) Die Endoprothesenteile (1 und 7) sind über eine konische Fläche (6, 8) miteinander verbindbar und weisen zwecks Veränderung der Halslänge eine zwischen diesen konischen Flächen schiebbare, konische Hülse (9) auf. Die Hülse weist eine Wandstärke von 1,5 mm auf, während sämtliche konische Flächen feine Längsrillen (10) aufweisen.

FIG.2

EP 0 011 665 A1

BEZEICHNUNG GEÄNDERT
siehe Titelseite

<u>Satz von Endoprothesenteilen</u>

Die vorliegende Erfindung bezieht sich auf einen Satz von Endoprothesenteilen, die über eine konische Fläche miteinander verbindbar sind.

Um eine Prothese, beispielsweise eine Hüft- oder Kniegelenkprothese, an den Körperbau und der erforderlichen Muskelspannung eines Patienten anpassen zu können, ist es wünschenswert, die Femurkomponente zweiteilig herzustellen, nämlich als Verankerungsstiel und Kugelkopf oder Kondylenteil, wobei letztere mit verschiedenen Halslängen gefertigt werden und einbringbar sind. Eine Vielzahl von Prothesenschäften, beispielsweise wie sie die Anmelderin herstellt und vertreibt und wie sie in der DE-OS 24 11 617 beschrieben sind, weisen am Prothesenschaft einen konischen Zapfen für die Fixierung des Kugelkopfes auf, der eine entsprechende konische Bohrung besitzt. Darauf ist eine einfache rotationsstabile Fixierung des Kopfes auf dem Schaftkonus möglich. Um die Länge eines solchen Prothesenschaftes anpassen zu können, werden Kugelköpfe mit verschiedenen Halslängen benötigt. Dies verteuert einerseits die Herstellung und andererseits die Lagerhaltung solcher Prothesenteile.

Es ist demgegenüber das Ziel der vorliegenden Erfindung, einen Satz von Prothesenteilen anzugeben, der es gestattet, die Halslänge des Prothesenschaftes auf einfache Weise dem Körperbau und der Muskelspannung des Patienten anzupassen.

Dieses Ziel wird dadurch erreicht, dass der Satz zwecks Veränderung der Halslänge der Endoprothese mindestens ein zwischen den konischen Flächen des Prothesenschaftes und des Kugelkopfes oder Kondylenteils schiebbares, konisches Einsatzstück aufweist.

Die Erfindung wird wird nun im einzelnen anhand einer Zeichnung von Ausführungsbeispielen näher erläutert werden.

Figur 1 zeigt, teilweise im Schnitt, den oberen Teil eines Prothesenschaftes für ein Hüftgelenk gemäss dem Stand der Technik,

Figur 2 zeigt, teilweise im Schnitt, als erstes Ausführungsbeispiel den oberen Teil eines Prothesenschaftes einer Hüftgelenkprothese und

Figur 3 zeigt, im Schnitt, als weiteres Ausführungsbeispiel einen Teil einer anderen Prothese.

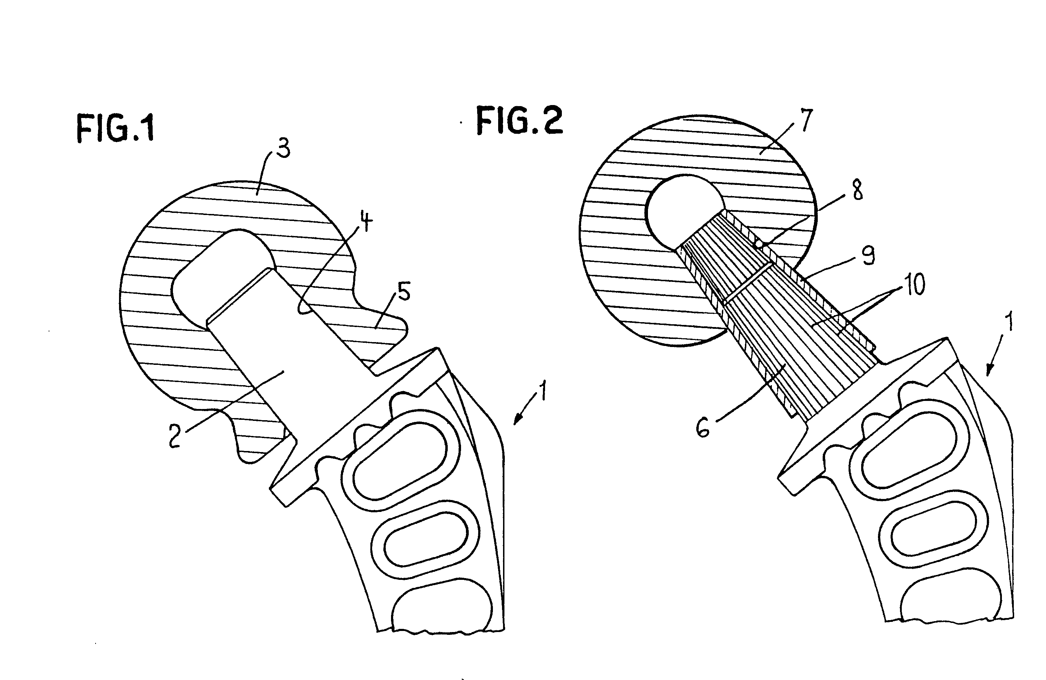

In Figur 1 erkennt man den Prothesenschaft 1 einer Hüftgelenkprothese mit dem konischen Zapfen 2, den Kugelkopf 3, vorzugsweise aus Keramik, mit der dem Zapfen entsprechenden Bohrung 4. Die übrigen Merkmale sind an sich bekannt und nicht erfindungswesentlich. Das Verjüngungsverhältnis des konischen Zapfen beträgt üblicherweise 1 : 10. Um die Halslänge des Prothesenschaftes dem Körperbau des Patienten anzupassen, werden gemäss dem Stand der Technik Kugelköpfe mit verschiedenen Halslängen 5 verwendet.

In Figur 2 ist ein erstes Ausführungsbeispiel dargestellt, wobei der Prothesenschaft 1 der gleiche ist wie in Figur 1. Der Zapfen 6 hingegen weist ein Verjüngungsverhältnis von 1 : 5 auf, während der Kugelkopf 7 aus Aluminiumoxyd-Keramik keinen Hals mehr aufweist und die Bohrung 8 die dem konischen Zapfen 6 entsprechende Konizität aufweist. Um die Halslänge dem Körperbau des Patienten anpassen

0011665

zu können, wird auf den Zapfen 6 eine konische Hülse 9 aufgeschoben. Zur Erhöhung der Drehstabilität und insbesondere zur Ermöglichung einer exakten
Einstellung der Position mit verschiedenen Rotationswinkeln, weisen sowohl der Zapfen 6 als auch beide Oberflächen der konischen Hülse und die Bohrung 8 des
Kugelkopfes feine Längsrillen 10 auf. Die Hülse
9 weist in diesem Beispiel eine Wandstärke von 1,5 mm
auf und ist aus einem geeigneten Metall hergestellt.
Zwecks Vermeidung einer Korrodierung der Metallhülse
kann es vorteilhaft sein, beide Oberflächen mit einer
Kunststoffschicht zu überziehen. Bei der Verwendung
einer Hülse von 1,5 mm Wandstärke mit einem Konus mit
einem Verjüngungsverhältnis von 1 : 5 lässt sich errechnen, dass durch das Aufsetzen einer solchen Hülse
die Halslänge um etwa 7,5 mm verlängert wird. Sollte
diese Verlängerung nicht ausreichen, kann eine zweite
Hülse über die erste geschoben werden. Es ist selbstverständlich auch möglich, Hülsen mit anderen unterschiedlichen Wandstärken herzustellen und zu verwenden
beispielsweise mit einer Wandstärke zwischen 1 und 4 mm.

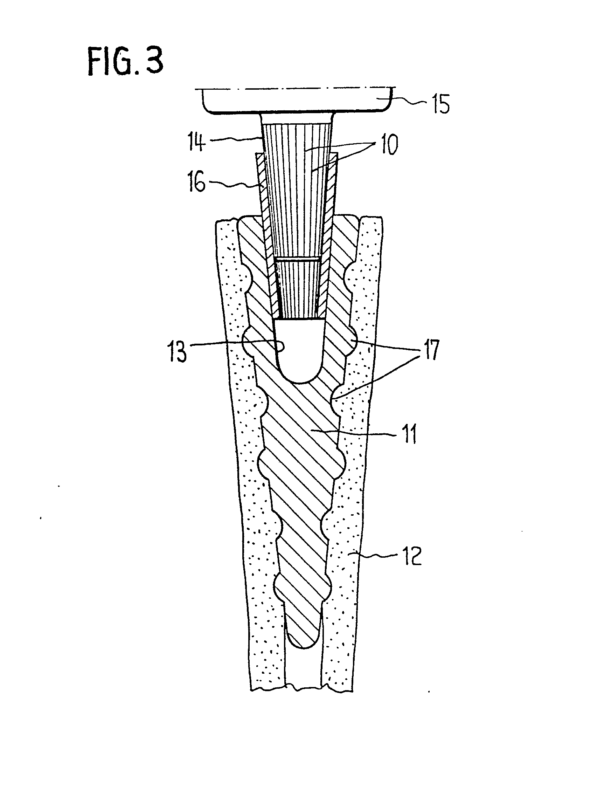

In Figur 3 ist ein Ausschnitt aus einer anderen Endoprothese dargestellt, beispielsweise eine Kniegelenkprothese. Man erkennt den Prothesenschaft 11 im Knochen

12, wobei der Prothesenschaft 11 Tragrippen 17 und eine konische Höhlung 13 aufweist, in die der Zapfen 14 des Kondylenteils 15 reicht. Auch hier beträgt das Verjüngungsverhältnis
des konischen Zapfens 1 : 5. Um die Halslänge des Prothesenschaftes zu verändern, wird ein ähnliches Einsatzstück 16 in Form einer konischen Hülse in die konische
Höhlung 13 eingeschoben, wobei selbstverständlich auch
hier zwei oder mehr Hülsen übereinander verwendet werden
können. Wie im vorhergehenden Fall weisen die konischen
Flächen     feine Längsrillen  auf.

Um die Herstellung eines solchen Einsatzstückes zu
erleichtern, kann es zweckmässig sein, ein geeignetes
Blech zu rollen und so ein aufgeschnittenes Einsatzstück zu erhalten. Auch solche Einsatzstücke können wie
die vorhergehend Beschriebenen mit einem Kunststoffüberzug versehen sein und     Längsrillen  aufweisen.

Die Erfindung ist selbstverständlich nicht auf Prothesenteile und konische Teile mit einem Verjüngungsverhältnis
von 1 : 5 beschränkt. Dieses Verhältnis kann beispielsweise auch 1 : 10 oder jeden anderen, die rotationsstabile Fixierung ermöglichenden Wert betragen.

Ausserdem bezieht sich die Erfindung auf Prothesenteile mit allen verwendbaren Materialien und Materialpaarungen, wie Metall, Kunststoff, faserverstärkter Kunststoff und Aluminiumoxyd-Keramik.

Patentansprüche

1. Satz von Endoprothesenteilen, die über eine konische
   Fläche miteinander verbindbar sind,

   dadurch gekennzeichnet,

   dass er zwecks Veränderung der Halslänge der Endoprothese mindestens ein zwischen den konischen Flächen (6, 8; 13, 14) des Prothesenschaftes (1, 11)
   und des Kugelkopfes (7) oder Kondylenteils (15)
   schiebbares, konisches Einsatzstück (9, 16) aufweist.

2. Satz gemäss Anspruch 1,

   dadurch gekennzeichnet,

   dass der Prothesenschaft (11) eine konische Höhlung
   (13) und das Kondylenteil (15) einen dazu passenden
   konischen Zapfen (14) aufweist.

3. Satz gemäss Anspruch 1 oder 2,

   dadurch gekennzeichnet,

dass das Verjüngungsverhältnis der konischen Teile
(6, 14) bei etwa 1 : 5 liegt.

4. Satz gemäss einem der Ansprüche 1 - 3,

   dadurch gekennzeichnet,

   dass die konischen Flächen feine Längsrillen (10)
   aufweisen.

5. Satz gemäss einem der Ansprüche 1 - 4,

   dadurch gekennzeichnet,

   dass das konische Einsatzstück (9, 16) eine Hülse
   mit einer gleichmässigen Wandstärke von 0,5 - 4 mm
   ist.

6. Satz gemäss Anspruch 5,

   dadurch gekennzeichnet,

   dass die Wandstärke 1,5 mm beträgt.

7. Satz gemäss Anspruch 5 oder 6,

   dadurch gekennzeichnet,

   dass die Oberflächen des konischen Einsatzstückes
   (9, 16) mit Kunststoff beschichtet sind.

8. Satz gemäss einem der Ansprüche 5 - 7,

   dadurch gekennzeichnet,

dass das konische Einsatzstück aufgeschnitten ist.


9. Verfahren zur Anpassung der Halslänge einer Endoprothese gemäss Anspruch 1 oder 2 an den Körperbau
eines Patienten,

dadurch gekennzeichnet,

dass mindestens ein konisches Einsatzstück entweder
über den konischen Zapfen oder in die konische Höhlung
des Prothesenschaftes geschoben wird.


10. Verfahren gemäss Anspruch 9,

dadurch gekennzeichnet,

dass zwei oder mehrere Einsatzstücke übereinander geschoben werden.

# FIG.1

# FIG.2

FIG.3

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | FR - A - 2 310 120 (CERAVER)<br><br>* Seite 2, Zeile 24 bis Seite 3, Zeile 8 *<br><br>------------ | 1 |

**KLASSIFIKATION DER ANMELDUNG (Int Cl ?)**

A 61 F 1/00

**RECHERCHIERTE SACHGEBIETE (Int. Cl.?)**

A 61 F

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 19-07-1979 | LEMERCIER |

EPA form 1503.1  06.78